# EUROPEAN PATENT SPECIFICATION

(11) **EP 3 515 510 B1**
(45) Date of publication and mention of the grant of the patent: **07.08.2024**
(21) Application number: 17777239.9
(22) Date of filing: 26.09.2017
(51) Int. Cl.: A61L 15/26, C08J 7/04, C08J 7/056

(54) **POLYURETHANE-HYDROGEL COMPOSITION COMPRISING CHLORHEXIDINE**
POLYURETHAN-HYDROGEL-ZUSAMMENSETZUNG MIT CHLORHEXIDIN
COMPOSITION DE POLYURÉTHANE-HYDROGEL COMPRENANT DE LA CHLORHEXIDINE

(30) Priority: 26.09.2016 IT 201600096247
(43) Date of publication of application: 31.07.2019
(73) Proprietor: Emodial S.r.l., 44124 Ferrara (IT)
(72) Inventor: VISMARA, Elena, 20157 Milan (IT); PECORARI, Federico, 44124 Ferrara (IT)
(74) Representative: Serravalle, Marco
(86) International application number: PCT/EP2017/074371
(87) International publication number: WO 2018/055200

(56) References cited:
- US-A1- 2005 033 251
- US-A1- 2013 131 621
- YIU-JIUAN LIN ET AL: "Stimulation of wound healing by PU/hydrogel composites containing fibroblast growth factor-2", JOURNAL OF MATERIALS CHEMISTRY B, vol. 3, no. 9, 1 January 2015 (2015-01-01), GB, pages 1931 - 1941, XP055697820, ISSN: 2050-750X, DOI: 10.1039/C4TB01638F
- BEATA BUTRUK ET AL: "Fabrication of biocompatible hydrogel coatings for implantable medical devices using Fenton-type reaction", MATERIALS SCIENCE AND ENGINEERING C, ELSEVIER SCIENCE S.A, CH, vol. 32, no. 6, 22 April 2012 (2012-04-22), pages 1601 - 1609, XP028519594, ISSN: 0928-4931, [retrieved on 20120428], DOI: 10.1016/J.MSEC.2012.04.050

## Description

The present invention is directed to a composition comprising a polyurethane covalently bond with a hydrogel forming polymer, and chlorexidine.

More particularly, the invention is directed to a composition comprising: a polyurethane substrate; a hydrogel forming polymer on at least one of the two faces of the polyurethane substrate; chlorexidine on the at least one face comprising the hydrogel forming polymer; wherein the hydrogel polymer is covalently bond to the polyurethane substrate and chlorexidine is preferably present in an amount comprised between 0.01 and 10.0 wt %, more preferably from 0.1 to 5 wt % based on the total weight of the composition. The composition has an optical transmittance higher than 30%.

It is well known that hydrogel dressings are particularly useful in the treatment of wounds due to their ability to absorb liquids released from the wound as well as keeping the wound wet. In fact, hydrogels are capable of both releasing and absorbing water.

It is also well known the use of chlorexidine in the treatment and prevention of infections due to its antibacterial properties.

EP 2 228 078 discloses a dressing consisting of a polyurethane support onto which a chitosan hydrogel is spread. The hydrogel phase further comprises a substance to aid healing, such as chlorexidine.

You-Jiuan Lin et al. ("Stimulation of wound healing by PU/hydrogel composites containing fibroblast growth factor-2", Journal of Material Chemistry B, vol. 3, n° 9, 1 January 2015, pages 1931-1941) disclose a wound dressing comprising a hydrogel grafted on a polyurethane porous substrate. Fibroblast growth factor-2 is impregnated in the hydrogel and the wound dressing possess antimicrobial activity.

The invention is as defined in the claims.

It has been surprisingly found that it is possible to prepare a transparent composition which is capable of adsorbing chlorexidine and release it in a controlled manner over time.

In a preferred embodiment, the composition according to the invention is used to prepare a transparent dressing. The transparent dressing comprises a polyurethane base and a hydrogel forming polymer covalently bond to the PU matrix. By having the hydrogel phase covalently bond to the matrix, the hydrogel phase does not remain on the skin, avoiding the unpleasant effect of having large amounts of hydrogel residues left on the skin after removal of the dressing.

The polyurethane (PU) used in the present invention can be chosen amongst a large variety of suitable polyurethanes. The term polyurethane indicates a polymer obtained by reaction of a polyisocianate with a polyol, e.g. a diisocianate with a diol. The PU used in the present invention is preferably a medical grade PU.

The polyisocyanate is preferably a diisocyanate, more preferably selected from toluendiisocyanate (TDI), 1,6-hexamethylene diisocyanate (HDI), isophorone diisocyanate (IPDI) and methylenediphenyl diisocyanate (MDI). Most preferably the polyisocyanate is methylenediphenyl diisocyanate.

The polyol used in the preparation of the PU is preferably an aliphatic polyol, or at least it is a polyol containing an aliphatic fraction. Examples of suitable polyols are ethylene glycol, dipropylene glycol, glycerol.

The surface of polyurethane is modified by binding a polymer capable of forming a hydrogel. The hydrogel forming polymer is PVP and more preferably a PVP having an average Mw comprised between 5,000 and 2,000,000, most preferably between 10,000 and 1,300,000.

The hydrogel forming polymer (PVP) is connected to PU through a free radical reaction preferably generated by a free radical initiator (e.g. cumene hydroperoxide) in the presence of a suitable initiator catalyst (e.g. Fe²⁺). The free radical generated on the initiator is capable of reacting with a suitable diolefin, e.g. ethylene glycol dimethacrylate (EGDMA). The free radical chain results in the hydrogel forming polymer to be covalently bond to PU. When using as a catalyst a metal salt, a reducing compound (e.g. ascorbic acid) is preferably present in the reaction medium to regenerate the initiator catalyst, e.g. to reduce Fe³⁺ to Fe²⁺. It is also possible to initiate the free radical chain by irradiating the solution with UV. In this case, the free radical initiator is preferably a diperoxide, e.g. dicumyl peroxide. The result is the formation of a hydrogel based on PVP and covalently bond to the surface of PU.

The amount of hydrogel-forming polymer deposited on the PU surface is preferably comprised between 1 and 50 wt %, depending on the used process conditions. More preferably the amount of polymer deposited on PU is comprised between 5 and 40 wt %, even more preferably between 8 and 30 wt %.

In one embodiment, the invention is directed to a process as defined in claim 6.

The amount of chlorhexidine deposited onto the PU-hydrogel is preferably comprised between 0.01 and 10 wt %, more preferably between 0.02 and 8 wt %, even more preferably between 0.05 and 5 wt %, or between 0.1 and 2.5 wt %. Any combination of the above values is also included in the present specifications.

Preferably, the PU-hydrogel composition is immerged in the chlorhexidine solution for a time comprised between 10 minutes and 12 h, more preferably between 30' and 6 h.

The concentration of chlorhexidine in the chlorhexidine solution can vary in a broad range, depending on the desired final concentration of chlorhexidine in the composition. Exemplary ranges of chlorhexidine concentration useful in the present invention are from 0.01 to 10 wt %, preferably from 0.1 to 5 wt%, more preferably from 0.2 to 3 wt %.

Chlorhexidine is not easily released once the dressing is placed on the wound, but it is slowly released in time, causing the dressing to be effective for at least one week without a significant decrease in chlorexidine concentration on the wound.

The dressing obtainable from the composition comprising chlorexidine according to the invention is characterized by a high efficacy as bacteriostatic agent and comprises Ag nanoparticles as additional bacteriostatic agent, in an amount in terms of kg of Ag nanoparticles per kilogram of composition preferably comprised between 1×10⁻⁵ and 2×10⁻², more preferably between 5×10⁻⁴ and 1×10⁻².

The silver salt used in the process according to claim 6, is preferably any water soluble silver salt, e.g. silver nitrate and silver fluoride. Preferably silver nitrate is used. The concentration of silver salt in the solution can vary in a very broad range depending on the amount of silver nanoparticles which is intended to deposit on the PU-hydrogel-chlorexidine substrate. In general, it varies between 0.01 M and 10.0 M, preferably between 0.02 M and 2.0 M, even more preferably between 0.05 M and 1.0 M.

Reduction of Ag⁺ to metallic silver is obtained either by chemically or physically induced reduction. For the chemical reduction, any suitable reducing agent can be used. Preferably the reducing agent is ascorbic acid, which can be preferably used in a concentration comprised between 0.1 and 10 w/v %, preferably from 0.2 to 5 w/v %. For physically induced reduction, a low pressure quicksilver UV lamp can be used.

The amount of silver impregnated in the PU-hydrogel-chlorhexidine can vary sensibly and in term of kg of silver per m² of composition is preferably comprised between 1×10⁻⁶ and 1×10⁻³ kg/m², more preferably between 5×10⁻⁶ and 5×10⁻⁴, even more preferably between 1×10⁻⁵ and 2×10⁻⁴.

In terms of weight percent based on the total weight of the composition or the dressing, the ratio is preferably comprised between 0.001% e 5.0 %, more preferably between 0.01% and 2.0%, even more preferably between 0.05% e 1.0%.

The volume average particle size of the silver nanoparticles is preferably comprised in the range from 20 nm to 500 nm, more preferably between 50 and 400 nm, even more preferably the volume average particle size of the silver nanoparticles is smaller than 300 nm, e.g. from 20 to 300 nm, or from 20 to 250 nm, or from 20 nm to 200 nm, or from 20 nm to 150 nm, as measured with conventional method for nanoparticles determination, e.g. by dynamic light scattering with a Malvern instrument.

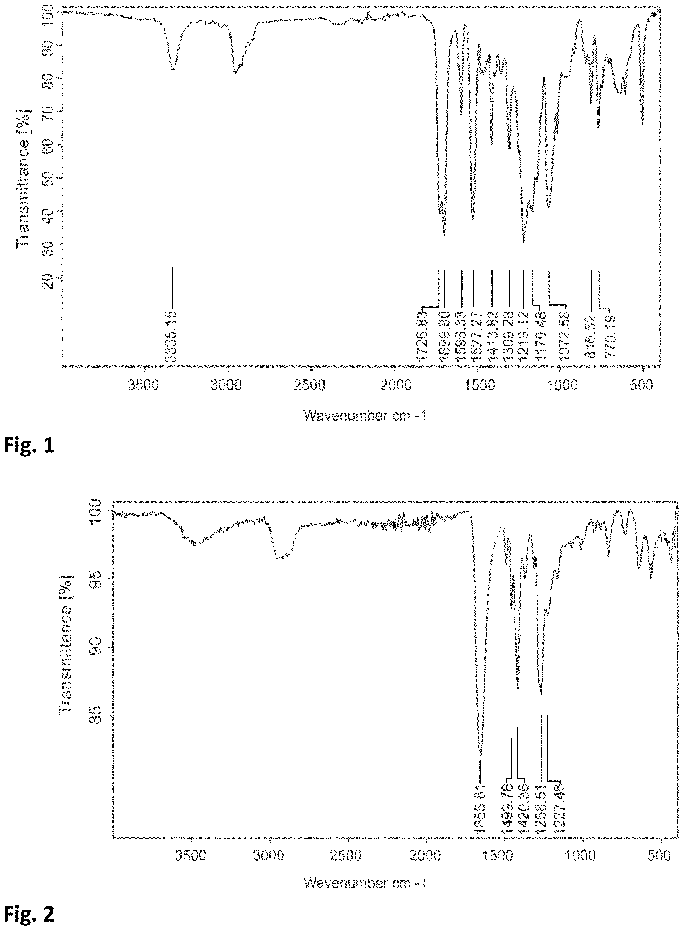

Figure 1 shows the NMR spectrum of polyurethane used in the tests.
Figure 2 shows the NMR spectrum of PVP.

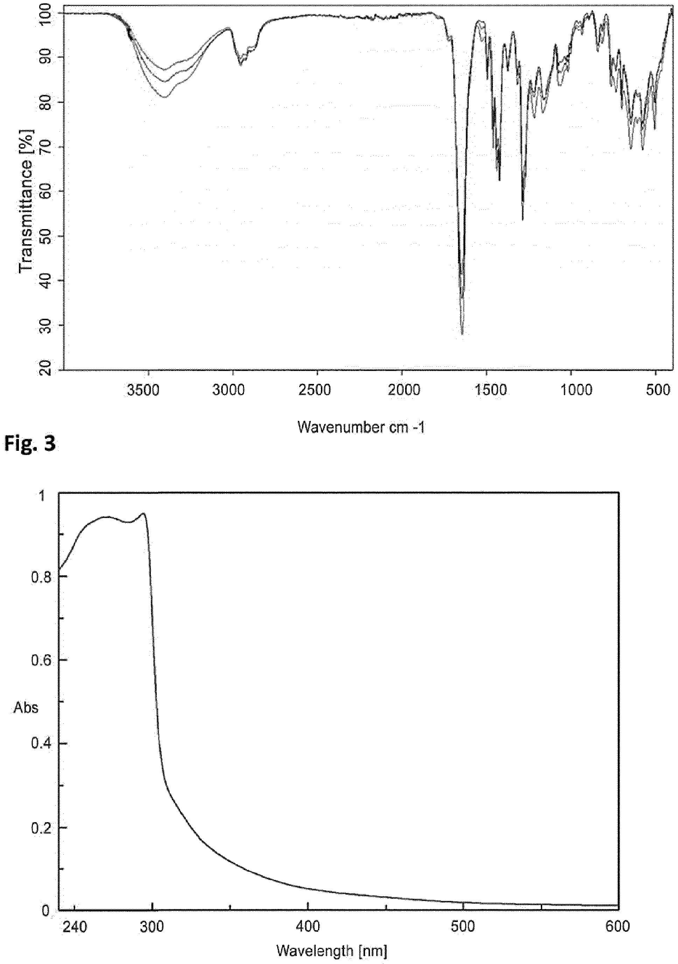

Figure 3 shows the NMR spectrum after formation of the hydrogel on the polyurethane sample. It is clear that most signals correspond to the signals of PVP, indicating that the surface of PU is uniformly covered by the PVP hydrogel.
Figure 4 shows the UV-Visible spectrum of PU covered with hydrogel. The very low absorbance between 300 and 600 nm indicates a very good optical transparency of the PU-hydrogel support.

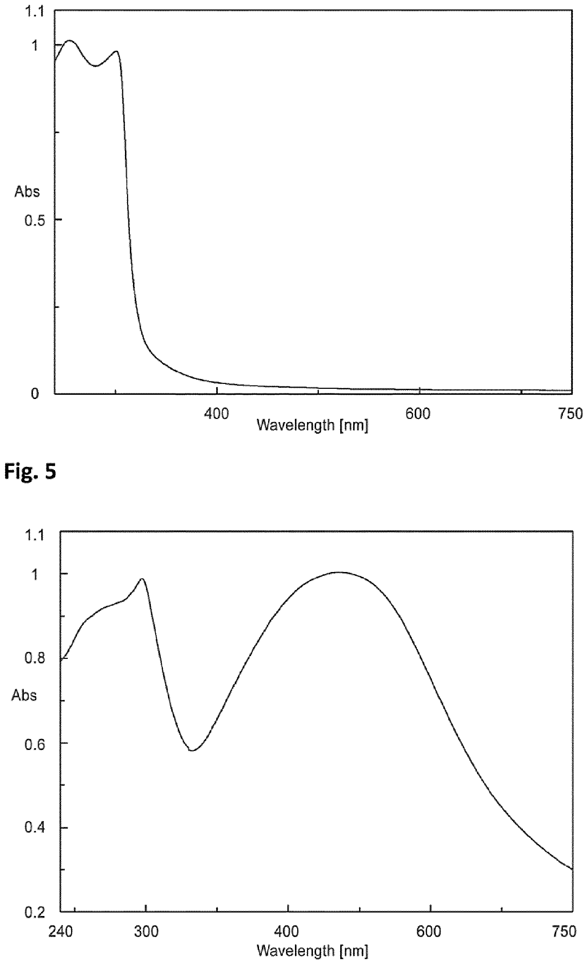

Figure 5 shows the UV-Visible spectrum of a PU-Hydrogel-Chlorexidine sample.
Figure 6 shows the UV-Visible spectrum of a PU-Hydrogel -Chlorexidine-Ag sample.

The dressing obtainable by the composition according to the invention can be used on skin wounds or at the entry port of a catheter implanted in a patient. The transparency of the dressing helps the visual check of the conditions of the skin underneath the dressing without removing the dressing itself.

Transparency of the dressing can be measured as optical transmittance according to spectroscopic UV-visible/Near IR analysis by using integrating spheres, which measure either the diffuse transmittance or reflectance of a liquid, solid or powder sample.

Typically, UV-Vis or UV-Vis/NIR spectrophotometers are used to measure the transmittance of a homogeneous, transparent liquid or solid sample. However, when a suspended liquid, opaque solid or powder is measured, the light incident to the sample is scattered and only a small portion of the diffusely transmitted or reflected light reaches the detector. An integrating sphere can acquire most of the light from the sample providing a more representative measurement of the sample.

The optical transmittance of the dressing according to the invention is higher than 30%, preferably higher than 50%, more preferably higher than 60%.

### Experimental

IR has been performed with the Alpha Bruker FT-IR spectrometer.

Ag has been measured by ICP-OES analysis with a Perkin Elmer Optima 8300.

UV-Vis/NIR spectrometry has been performed with a Jasco V-700 Series UV-Visible/NIR spectrophotometer equipped with a 60mm UV-Visible/NIR Integrating Spheres.

The following reagents were used: Chlorexidine, 20% w/v non-sterile solution from Alfa Aesar; ascorbic acid from Sigma Aldrich reagent grade; sodium dodecyl sulfate from Sigma Aldrich, purity >98%; PVP from Sigma Aldrich, with average Mw of 360.000 Da.

The examples according to the invention are only the examples comprising a composition comprising: a. a polyurethane substrate;

b. a polyvinylpyrrolidone (PVP) hydrogel on at least one of the two faces of the polyurethane substrate;

c. chlorhexidine and Ag nanoparticles on the at least one face comprising the PVP hydrogel; wherein the PVP hydrogel is covalently linked to the polyurethane substrate.

### Preparation of a PU modified with a gel-forming polymer

A polyurethane coated with PVP was prepared according to the method described by B. Butruk et al. "Fabrication of biocompatible hydrogel coatings for implantable medical devices using Fenton-type reaction" Material Science and Engineering C32 (2012) 1601-1609, whose content is herein incorporated by reference. A sample of polyurethane (PU) medical grade was immersed in a toluene solution containing EGDMA (10 % by volume) and cumene hydroperoxide (CPH, 8 % by volume). The sample was left in the solution for 5 minutes without stirring, to allow CPH and EGDMA to diffuse into the PU matrix. The sample was then extracted from the solution and dried in air for about 1 min and then immersed in an aqueous solution containing FeCl₂x4H₂O (0.1 % w/v), ascorbic acid (AA, 1% w/v), and PVP 360 (5% w/v) for 15 minutes, stirring every 3-4 minutes manually. The sample was then washed abundantly and under magnetic stirring with the following solutions: aqueous solution of sodium dodecyl sulfate (0.1% w/v), a PBS (phosphate buffer saline) solution, a solution H₂O/ethanol 60/40 (v/v), 2 washings with deionized water.

After the washings, the sample (PUH1) was left to dry in air, till a constant weight is reached (approx. 12 hours). The samples were weighted and by difference between the initial weight (0.5350 g) and the final weight (0.6094 g) it was calculated that the hydrogel was present on the PU in an amount of 0.0744 g, i.e. 14 wt % with respect to PU.

The procedure was repeated on a smaller PU sample (0.2568 g) leading to a PU-hydrogel sample (PUH2) of 0.2982 g containing an amount of hydrogel of 0.0414 g, equal to 16 wt % with respect to PU.

### Deposition of Chlorhexidine on PU-Hydrogel

Two samples of PUH1 and PUH2 were charged with chlorhexidine by immerging the samples in a 5 wt % solution of chlorhexidine digluconate. After drying, the sample contained 4.8 wt % (PHU1) and 12.1 wt % (PHU2) of chlorhexidine.

However, part of chlorhexidine is not fixed onto the substrate in a stable way. In fact, when immerging the dried composition in water, it was noticed that a considerable amount of chlorhexidine is dissolved in water and the amount left on PU-hydrogel is respectively 2.74 wt % and 2.36 wt %. Table 1 shows the kinetic of release of chlorhexidine from PUH1. The kinetic was followed by UV analysis and the amount released measured by gravimetric analysis.

**Table 1**

| Kinetic of release of chlorexidine | | |
|---|---|---|
| Time (min) | Abs (254 nm) | mg released |
| 0 | 0 | |
| 5 | 0.3839 | |
| 10 | 0.5257 | 10.98 |
| 20 | 0.4735 | |
| 30 | 0.4628 | |
| 45 | 0.4414 | |
| 60 | 0.4413 | |
| 120 | 0.4205 | |
| 180 | 0.4210 | |
| 900 | 0.4112 | |
| 1500 | 0.3994 | |
| 5760 | 0.4195 | |
| 7200 | 0.4160 | 8.57 |
| 8640 | 0.3903 | |

As it is clear from table 1, there is equilibrium between chlorhexidine dissolved and adsorbed in the PU-hydrogel and the amount measured in solution does not considerably change in time even when considering a period of 5 days.

### Deposition of Ag nanoparticles on PU-hydrogel-chlorexidine

Samples of about 200 mg PU-hydrogel-chlorexidine (30 wt % chlorexidine) were immersed in 100 mL solution of 0.05 M AgNO₃ for 24 hours. The Ag salt deposited on the sample was fixed onto it by reduction to metallic silver by 30' irradiation with low pressure quicksilver lamp. Large part of chlorexidine was recovered in the AgNO₃ solution. (Ag= 0.485 mg/100 mg or 2.19 mg/100 cm²; chlorexidine 0.155-0,21 mg/100 mg or 0.7-0.95 mg/100 cm²)

### Bacteriostatic activity of the PU-Hydrogel- Ag-chlorexidine dressing

The bacteriostatic activity of the dressing comprising chlorhexidine and Ag nanoparticles was tested according ASTM E 2180 -01. Samples of the PU-Hydrogel-chlorexidine-Ag- dressing having an area of the surface of 9 cm² where prepared in triplicate for each test. A thin layer of agar, previously contaminated, was distributed on each sample. Immediately after contamination and after defined contact times (24h, 48h, 4 days and 7 days) the microorganisms were collected by using a neutralizing nutrient solution. The various collected solutions were serially diluted, sown in double and incubated for 48/72 h at 37 °C. Results are expressed as geometric mean and as percentage of reduction of the microorganisms with respect to the starting amount. The results indicate a bacteriostatic activity of 100% with respect to all strains tested.

| | Staphylococcus epidermidis | Staphylococcus aereus | Candida albicans | Pseudomonas aeruginosa |
|---|---|---|---|---|
| Starting inoculus | 4.3×10⁷ Ufc/g | 2.6×10⁷ Ufc/g | 4.3×10⁷ Ufc/g | 8.0×10⁷ Ufc/g |
| T 24 h | <1 | <1 | <1 | <1 |
| T 48 h | <1 | <1 | <1 | <1 |
| T 4 days | <1 | <1 | <1 | <1 |
| T 7 days | <1 | <1 | <1 | <1 |

## Claims

1. A composition comprising:
a. a polyurethane substrate;
b. a polyvinylpyrrolidone (PVP) hydrogel on at least one of the two faces of the polyurethane substrate;
c. chlorhexidine and Ag nanoparticles on the at least one face comprising the PVP hydrogel;
wherein the PVP hydrogel is covalently linked to the polyurethane substrate.

2. The composition according to claim 1 wherein chlorhexidine present in an amount comprised between 0.01 and 10.0 wt % based on the total weight of the composition.

3. The composition according to claims 1-3, wherein the PVP hydrogel is present in an amount comprised between 1 and 50 wt % based on the amount of polyurethane plus PVP hydrogel.

4. The composition of any of claims 1-3, wherein the amount of silver nanoparticles impregnated in the composition is comprised between 1×10⁻⁶ and 1×10⁻³ kg/m².

5. A dressing comprising the composition of any of claims 1-4.

6. A process for the preparation of a composition according to claims 1-4, the process comprising the following steps:
i) impregnating at least one face of a polyurethane substrate with a solution comprising a reactive diolefin and a free radical initiator;
ii) extracting the polyurethane from the solution and, after drying, immerging the polyurethane in a solution containing the initiator catalyst and PVP to obtain PU covalently bond to PVP;
iii) extracting the polyurethane-PVP composition and drying in air;
iv) immerging the product of step iii) into an aqueous solution of chlorhexidine;
v) collecting the polyurethane-PVP hydrogel-chlorhexidine product from the solution and drying the obtained composition in air
vi) depositing silver salts by immerging the polyurethane-PVP hydrogel-chlorhexidine product in a solution of the silver salt, followed by reduction by chemical or physical methods; and
vii) collecting the product of step vi) and drying it in air.

7. The process according to claim 6 wherein the physical methods comprise UV irradiation with low pressure quicksilver lamps, and the chemical methods comprise treatment an aqueous solution of ascorbic acid.

## Patentansprüche

1. Zusammensetzung, die Folgendes umfasst:
a. ein Polyurethan-Substrat;
b. ein Polyvinylpyrrolidon (PVP)-Hydrogel auf mindestens einer der beiden Seiten des Polyurethan-Substrats;
c. Chlorhexidin und Ag-Nanopartikel auf der mindestens einen Seite, die das PVP-Hydrogel umfasst;
wobei das PVP-Hydrogel kovalent mit dem Polyurethansubstrat verbunden ist.

2. Zusammensetzung nach Anspruch 1, wobei Chlorhexidin in einer Menge zwischen 0,01 und 10,0 Gew.-%, bezogen auf das Gesamtgewicht der Zusammensetzung, vorliegt.

3. Zusammensetzung nach Anspruch 1 bis 3, wobei das PVP-Hydrogel in einer Menge zwischen 1 und 50 Gew.-%, bezogen auf die Menge an Polyurethan plus PVP-Hydrogel, vorhanden ist.

4. Zusammensetzung nach einem der Ansprüche 1 bis 3, wobei die Menge der in der Zusammensetzung imprägnierten Silbernanopartikel zwischen 1×10⁻⁶ und 1×10⁻³ kg/m² liegt.

5. Verband, umfassend die Zusammensetzung nach einem der Ansprüche 1-4.

6. Verfahren zur Herstellung einer Zusammensetzung nach einem der Ansprüche 1-4, wobei das Verfahren die folgenden Schritte umfasst:
i) Imprägnieren mindestens einer Seite eines Polyurethansubstrats mit einer Lösung, die ein reaktives Diolefin und einen Radikalinitiator enthält;
ii) Extrahieren des Polyurethans aus der Lösung und, nach dem Trocknen, Eintauchen des Polyurethans in eine Lösung, die den Initiator-Katalysator und PVP enthält, um PU zu erhalten, das kovalent an PVP gebunden ist;
iii) Extrahieren der Polyurethan-PVP-Zusammensetzung und Trocknen an der Luft;
iv) Eintauchen des Produkts aus Schritt iii) in eine wässrige Lösung von Chlorhexidin;
v) Sammeln des Polyurethan-PVP-Hydrogel-Chlorhexidin-Produkts aus der Lösung und Trocknen der erhaltenen Zusammensetzung an der Luft;
vi) Abscheidung von Silbersalzen durch Eintauchen des Polyurethan-PVP-Hydrogel-Chlorhexidin-Produkts in eine Lösung des Silbersalzes, gefolgt von einer Reduktion durch chemische oder physikalische Verfahren; und
vii) Sammeln des Produkts aus Schritt vi) und Trocknen an der Luft.

7. Verfahren nach Anspruch 6, wobei die physikalischen Methoden UV-Bestrahlung mit Niederdruck-Quicksilberlampen umfassen und die chemischen Methoden die Behandlung einer wässrigen Lösung von Ascorbinsäure umfassen.

## Revendications

1. Composition comprenant :
a. un substrat en polyuréthane ;
b. un hydrogel de polyvinylpyrrolidone (PVP) sur au moins une des deux faces du substrat en polyuréthane ;
c. des nanoparticules de chlorhexidine et d'Ag sur l'au moins une face comprenant l'hydrogel de PVP ;
dans lequel l'hydrogel de PVP est lié de manière covalente au substrat en polyuréthane.

2. Composition selon la revendication 1, dans laquelle la chlorhexidine est présente en une quantité comprise entre 0,01 et 10,0 % en poids par rapport au poids total de la composition.

3. Composition selon les revendications 1 à 3, dans laquelle l'hydrogel de PVP est présent en une quantité comprise entre 1 et 50 % en poids sur la base de la quantité de polyuréthane plus l'hydrogel de PVP.

4. Composition selon l'une quelconque des revendications 1 à 3, dans laquelle la quantité de nanoparticules d'argent imprégnées dans la composition est comprise entre 1×10⁻⁶ et 1×10⁻³ kg/m².

5. Pansement comprenant la composition selon l'une quelconque des revendications 1 à 4.

6. Procédé pour la préparation d'une composition selon les revendications 1 à 4, le procédé comprenant les étapes suivantes :
i) imprégnation d'au moins une face d'un substrat en polyuréthane avec une solution comprenant une dioléfine réactive et un initiateur de radicaux libres ;
ii) extraction du polyuréthane de la solution et, après séchage, immersion du polyuréthane dans une solution contenant le catalyseur initiateur et le PVP pour obtenir une liaison covalente du PU au PVP ;
iii) extraction de la composition polyuréthane-PVP et séchage à l'air ;
iv) immersion du produit de l'étape iii) dans une solution aqueuse de chlorhexidine ;
v) collecte du produit hydrogel de polyuréthane-PVP-chlorhexidine de la solution et séchage de la composition obtenue à l'air
vi) dépôt des sels d'argent en immergeant le produit d'hydrogel-chlorhexidine de polyuréthane-PVP dans une solution du sel d'argent, suivie d'une réduction par des procédés chimiques ou physiques ; et
vii) collecte du produit de l'étape vi) et le séchage à l'air.

7. Procédé selon la revendication 6, dans lequel les procédés physiques comprennent l'irradiation UV avec des lampes à vif-argent à basse pression, et les procédés chimiques comprennent le traitement d'une solution aqueuse d'acide ascorbique.
